# EUROPEAN PATENT SPECIFICATION

(11) **EP 0 779 264 B1**
(45) Date of publication and mention of the grant of the patent: **16.08.2000**
(21) Application number: 96119656.5
(22) Date of filing: 07.12.1996
(51) Int. Cl.: C07C 49/675, C07C 45/36

(54) **Method for production of fluorenone**
Verfahren zur Herstellung von Fluorenon
Procédé pour la préparation d'acétone

(30) Priority: 11.12.1995 JP 32137095; 11.12.1995 JP 32137195
(43) Date of publication of application: 18.06.1997
(60) Divisional application: 99123813.0
(73) Proprietor: NIPPON SHOKUBAI CO., LTD., Chuo-ku, Osaka-shi, Osaka-fu 541 (JP)
(72) Inventor: Tsukasa, Takahashi, Himeji-shi, Hyogo-ken (JP); Yasuhisa, Emoto, Ibo-gun, Hyogo-ken (JP)
(74) Representative: Luderschmidt, Schüler & Partner GbR

(56) References cited:
- US-A- 1 374 695
- US-A- 1 764 023
- PATENT ABSTRACTS OF JAPAN vol. 010, no. 103 (C-340), 18 April 1986 & JP 60 233028 A (NIPPON SHOKUBAI KAGAKU KOGYO KK), 19 November 1985,

## Description

This invention relates to a method for the production of fluorenone and more particularly to a method for producing fluorenone by the vapor-phase catalytic oxidation of fluorene with a high yield. Fluorenone is a commercially useful substance as raw materials for agricultural pesticides, medicines, and functional macromolecular compounds.

### Description of the Prior Art:

The method for producing fluorenone by the vapor-phase catalytic oxidation of fluorene with molecular oxygen is at an advantage in enjoying high productivity as compared with the liquid-phase method and avoiding emission of waste liquor. It is nevertheless at a disadvantage in having to sacrifice selectivity when the reaction is performed at a high conversion. It incurs difficulty in effectively utilizing fluorene while maintaining high productivity.

The method for the production of fluorenone is known in two types, the liquid-phase method and the vapor-phase method. As to the liquid-phase method, many reports have been published on the method of liquid-phase oxidation with molecular oxygen by the use of a phase-transfer catalyst formed of an aqueous alkali solution, a hydrophobic organic solvent, and a quaternary ammonium salt (JP-A-07-82, 206 and JP-A-07-82,207). As to the vapor-phase method, a method using vanadium pentoxide (U.S. Patent No. 1,374,695), a method using a catalyst formed of iron vanadate and potassium sulfate [Zh. Pyirl Khim 35, 693-696 (1962)], a method using a catalyst formed of vanadium pentoxide and tin oxide [Engineering Chemical Journal, 56, (6), 413-416 (1953)], a method for causing inclusion of a large volume of water by the use of a catalyst formed of metal salts and alkali metal sulfates of vanadic acid, molybdic acid, or tungstic acid (U.S. Patent No. 1,892,768), a method using a catalyst formed of vanadium pentoxide, silica, and potassium sulfate (U.S. Patent No. 2,956,065), and a method using a catalyst formed of vanadium, titania, and an alkali metal (JP-A-60-233,028) have been disclosed. In addition to these methods, a method using a catalyst formed of vanadium, iron, and cesium [Stud, Surf, Sci. Catal. (1993), 75 (New Frontiers in Catalysis, Pt. A), 707-17] has been also disclosed.

It has been heretofore known that a vanadium-based catalyst can be used as an effective catalyst for the production of fluorenone by the vapor-phase oxidation of fluorene. It has been difficult, however, to obtain fluorenone stably with a high yield because the reaction performed at a high conversion results in lowering the selectivity.

An object of this invention, therefore, is to provide an improved method for the production of fluorenone.

Another object of this invention is to solve the problems heretofore attendant on the vapor-phase oxidation and provide a method for producing fluorenone 'with high selectivity.

### SUMMARY OF THE INVENTION

The objects mentioned above are accomplished, in the production of fluorenone by the vapor-phase catalytic oxidation of fluorene with a molecular oxygen-containing gas, by a method for the production of fluorenone which comprises adjusting the molar ratio of fluorene/molecular oxygen in a feed raw material gas consisting of fluorene as a raw material and a molecular oxygen-containing gas in the range of 1 to 0.13. This range, when air is used as the molecular oxygen-containing gas, corresponds approximately to a fluorene content in the range of 200 to 1280 g per 1 Nm³ of the feed raw material gas.

Our study has evolved a knowledge that in the vapor-phase catalytic oxidation of fluorene with a molecular oxygen-containing gas, the fluorenone is obtained at a high conversion with high selectivity by adjusting the fluorene concentration in the feed raw material gas consisting of fluorene as a raw material and the molecular oxygen-containing gas within a range higher than that of a conventional method. This invention has been perfected as a result of this knowledge.

According to this invention, fluorenone can be produced at a high conversion with high selectivityby the vapor-phase catalytic oxidation of fluorene. Particularly, this invention accomplishes the high selectivity with the conversion of fluorene kept at such a high level as that approximating closely to 100%.

### BRIEF DESCRIPTION OF THE DRAWINGS

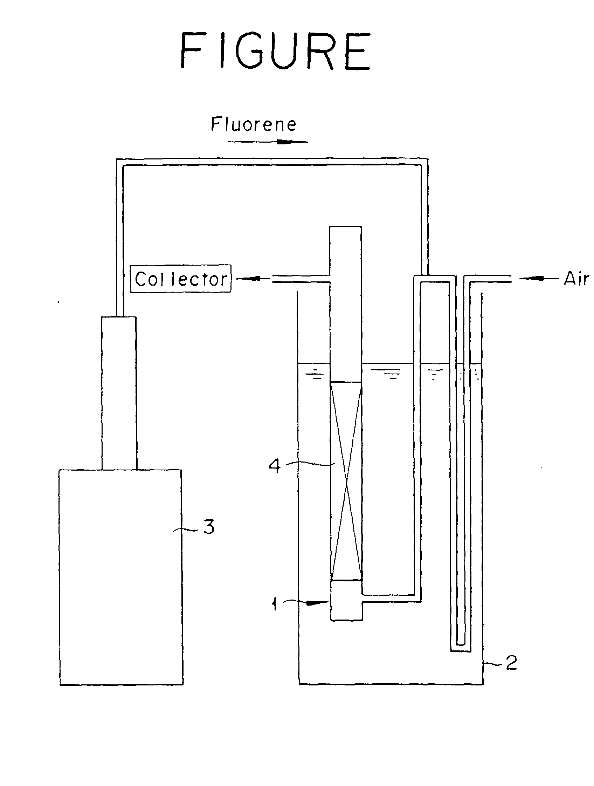

Fig. 1 is a schematic diagram of a reaction apparatus to be used in working examples and controls herein.

### DESCRIPTION OF THE PREFERRED EMBODIMENT

The fluorene to be used as the raw material in this invention may be any of various species of fluorene such as, for example, the fluorene which is obtained from a fluorene-containing oil derived from coal tar (tar type fluorene) and the fluorene which is obtained from the residual oil by-produced in the process for the production of dealkylated benzene (petroleum process type fluorene) . The raw material fluorene does not need to completely consist of fluorene but may contain various impurities arising from the starting raw material or the process of purification such as, for example, biphenyl compounds like methyl biphenyl, naphthalene compounds like methyl naphthalene, fluorene derivatives like 9-methyl fluorene, and dibenzofuran.

Even when the fluorene concentration in the raw material fluorene is not more than 60% by weight, the production of fluorenone may be obtained with a high yield. In consideration of the necessity for a subsequent step for purification, however, it is appropriate to use the raw material fluorene having a fluorene concentration of not less than 70% by weight, preferably not less than 80% by weight.

The catalyst to be used for the vapor-phase catalytic reaction according to this invention is not particularly limited. Any of the catalysts that are generally used for the vapor-phase oxidation of fluorene into fluorenone can be used. As concrete examples of the catalyst which is effectively usable herein, a catalyst formed of vanadium pentoxide, a catalyst formed of iron vanadate and potassium sulfate, a catalyst formed of vanadium pentoxide and tin oxide, a catalyst formed of a metal salt or an alkali metal sulfate of vanadic acid, molybdic acid, or tungstic acid, a catalyst formed of vanadium pentoxide, silica, and potassium sulfate, a catalyst formed of vanadium, titania, and an alkali metal, and a catalyst formed of vanadium, iron, and cesium sulfate maybe cited. Among other catalysts mentioned above, the catalyst formed of vanadium, titanium oxide, and an alkali metal sulfate is used particularly advantageously. Here, the atomic ratio of alkali metal element/vanadium appropriately falls in the range of 0.8 to 8, preferably 1 to 5, particularly preferably 1 to 3. It is particularly advantageous to use a supported catalyst having such a catalytically active component as mentioned above deposited on such an inert carrier as of silicon carbide, alumina, silica, silica-alumina, or pumice. Incidentally, the carried catalyst is in the form of spheres, cylinders, saddles, or cylindricalparticles which generally have a particle diameter in the range of 3 to 15 mm, preferably 3 to 10 mm.

One of the characteristics of this invention, in the oxidation to be effected by causing a feed raw material gas consisting of fluorene and a molecular oxygen-containing gas to contact with the catalyst mentioned above, resides in adjusting the molar ratio of fluorene/molecular oxygen in the feed raw material gas in the range of 1 to 0.13, preferably 0.9 to 0.3, and most preferably 0.8 to 0.6 . If the molar ratio of the fluorene/molecular oxygen in the feed raw material gas is more than 1, oxygen becomes insufficient and the conversion of fluorene is lowered rapidly. The above-mentioned molar ratios, when air is used as the molecular oxygen-containing gas, correspond to the fluorene contents per 1 Nm³ of the feed raw material gas in the range of 200 to 1280 g/Nm³, 450 to 1170 g/Nm³, and 900 to 1060 g/Nm³, respectively.

In the context of this invention, the term "molar ratio of fluorene/molecular oxygen" is defined as a molar ratio of (moles of pure fluorene) / [(moles of molecular oxygen in the raw material gas) - (moles of oxygen to be consumed by impurities)]. Examples of the molecular oxygen-containing gas include normally air, as well as oxygen-rich air, and a mixed gas of air with such an inert gas as nitrogen and carbon dioxide . Further, the raw material gas, when necessary, may be used as diluted with steam.

Even when pure oxygen is used as the molecular oxygen-containing gas in the method of this invention, the fluorene concentration in the feed raw material gas does not exceed 3710 g/Nm³ because of the requirement that the molar ratio of fluorene/molecular oxygen should be in the range of 1 to 0.13. It is thought appropriate to use normally air as a molecular oxygen-containing gas from the economic point of view. In this case, the fluorene concentration in the feed raw material gas does not exceed 1280 g/Nm³.

The reaction conditions such as the temperature, the spatial velocity, and the like to be involved in the vapor-phase catalytic oxidation contemplated by this invention are not limited particularly but may be suitably selected, depending on the kind of the catalyst to be used, for example.

Generally, the reaction temperature is selected in the range of 250° to 480 ° C, preferably 300° to 450 ° C and the spatial velocity in the range of 100 to 10,000/hr, preferably 200 to 5,000/hr.

Now, this invention will be described more specifically below with reference to working examples.

### Preparation Example 1

A homogenous solution was obtained by stirring 13.4 g of ammonium vanadate, 26.8 g of oxalic acid, 2.4 g of potassium sulfate, and 25.1 g of cesium sulfate in 180 ml of pure water at 80°C. This solution was cooled to room temperature. A homogeneous slurry was formed by thoroughly stirring 120 g of anatase type titanium dioxide having a surface area of 20 m²/g and 4.8 g of silicon carbide whiskers in the cooled solution. A slurry for the preparationof catalyst was obtained by adding 400 ml of pure water to the resultant slurry.

A stainless steel heatable rotary drum was charged with 200 g of beads of silicon carbide carrier of 4 mm in average diameter, and set rotating. The silicon carbide carrier in the rotating kiln was kept at a temperature in the range of 180° to 220 ° C and 20 g of the slurry for the preparation of catalyst mentioned above was sprayed and deposited on the beads of silicon carbide carrier. The beads now having the catalyst carried thereon were calcined as swept with a stream of air at 550 ° C for five hours, to prepare an oxidizing catalyst. The atomic ratio of each added element was as follows.
V : Cs : K : S : TiO₂ = 7.64 : 9.26 : 1.84 : 5.55 : 100

### Control 1

In a stainless steel reaction tube 1 of 20 mm in inside diameter, the oxidizing catalyst which was obtained in the above Preparation Example 1 was packed in a bed length of 180 mm on the gas outlet side of the reaction tube as shown in Fig. 1. This reaction tube 1 was retained in a molten salt bath 2 at 430 ° C. In the apparatus illustrated in Fig. 1, a raw material fluorene (fluorene concentration 98% by weight) originating in a petroleum process and fed from a feeder 3 via a pipe kept at 140 ° C was forwarded to an inlet part of the reaction tube and introduced as mixed with preheated air into a catalyst bed 4. The reaction of fluorene and air was carried out with the feed rate of fluorene fixed at 9.59 mg/minute (as pure fluorene) and the air flow fixed at 500 ml/minute (at 0 ° C under one atmosphere) . The fluorene concentration in the feed raw material gas at this time was 19.1 g per 1 Nm³ of feed raw material gas.

The gas produced by the reaction was collected via an air-cooled glass collecting tube of 35 mm in inside diameter (not shown) and three aerating vials (not shown) arranged in series and filled with acetone. It was recovered in the form of an acetone solution and then analyzed with a gas chromatography using a column OV-1/0.25 mm ID 50 m (produced by Shimadzu Seisakusho, Ltd. and marketed under product code of "GC-14B"). Consequently, the conversion of fluorene was found to be 99.2 mol%, the selectivity of fluorenone to be 87.4 mol%, and the yield of fluorenone to be 86.8 mol%.

### Examples 1 to 4

The oxidation reaction was carried out by following the procedure of Control 1 while adjusting the fluorene concentration in the feed raw material gas to 253 g/Nm³ (Example 1), 482 g/Nm³ (Example 2) , 901 g/Nm³ (Example 3), and 1031 g/Nm³ (Example 4) by varying the feed rate of fluorene. The results are shown in Table 1.

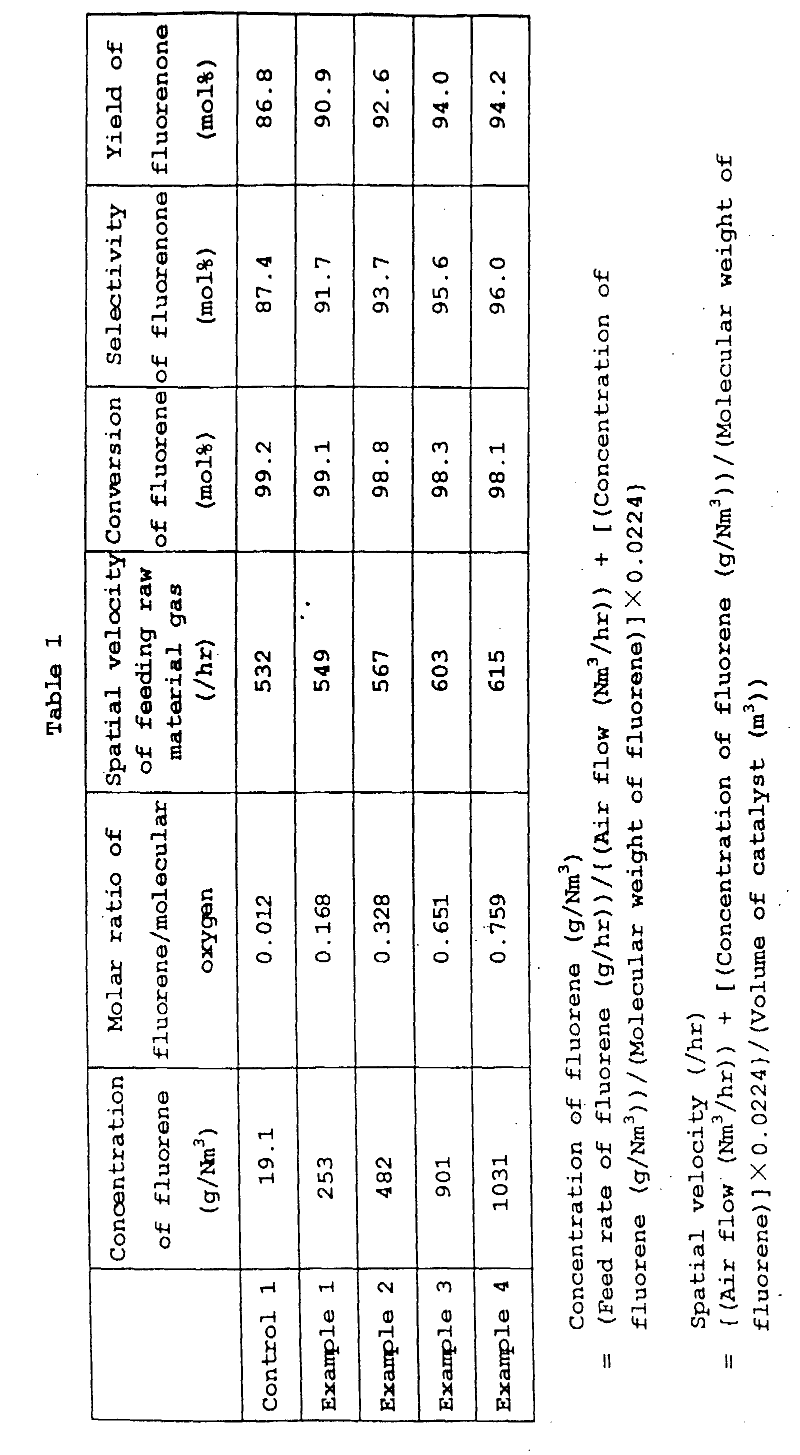

## Claims

1. A method for the production of fluorenone by the vapor-phase catalytic oxidation of fluorene with a molecular oxygen-containing gas, which comprises adjusting the molar ratio of fluorene/molecular oxygen in a feed raw material gas consisting of fluorene as a raw material and a molecular oxygen-containing gas in the range of 1 to 0.13.

2. A method according to claim 1, wherein said molecular oxygen-containing gas is air.

3. A method according to claim 1, wherein said catalyst contains at least vanadium, titania, and an alkali metal and the atomic ratio of said alkali metal to vanadium is in the range of 0.8 to 8.

## Patentansprüche

1. Verfahren zur Herstellung von Fluorenon durch katalytische Dampfphasenoxidation von Fluoren mit einem molekularen Sauerstoff enthaltenden Gas, umfassend die Einstellung des Molverhältnisses von Fluoren zum molekularen Sauerstoff in einem gasförmigen Beschickungsausgangsmaterial, das aus Fluoren als Ausgangsmaterial und einem molekularem Sauerstoff enthaltenden Gas besteht, in dem Bereich von 1 bis 0,13.

2. Verfahren gemäß Anspruch 1, bei dem das molekularen Sauerstoff enthaltende Gas Luft ist.

3. Verfahren gemäß Anspruch 1, bei dem der Katalysator zumindest Vanadium, Titandioxid und ein Alkalimetall enthält, und das Atomverhältnis von Alkalimetall zu Vanadium im Bereich von 0,8 bis 8 liegt.

## Revendications

1. Procédé pour la production de fluorénone par l'oxydation catalytique en phase vapeur de fluorène avec un gaz contenant de l'oxygène moléculaire, procédé qui comprend l'ajustement du rapport molaire entre le fluorène/l'oxygène moléculaire dans un gaz de matière première d'alimentation constitué par le fluorène comme matière première et un gaz contenant de l'oxygène moléculaire dans la plage de 1 à 0,13.

2. Procédé selon la revendication 1, dans lequel ledit gaz contenant de l'oxygène moléculaire est de l'air.

3. Procédé selon la revendication 1, dans lequel ledit catalyseur contient au moins de vanadium, de l'oxyde de titane, et un métal alcalin et le rapport atomique entre le métal alcalin et le vanadium se situe dans la plage de 0,8 à 8.
